(19) Europäisches Patentamt
European Patent Office
Office européen des brevets

(11) Publication number: **0 237 080 B1**

(12) **EUROPEAN PATENT SPECIFICATION**

(45) Date of publication of patent specification: **03.06.92**

(51) Int. Cl.⁵: **C12P 17/02**, C12P 41/00

(21) Application number: **87103683.6**

(22) Date of filing: **13.03.87**

(54) **Process for the preparation of L(-)-carnitine chloride starting from 3,4-epoxybutyric esters.**

(30) Priority: **14.03.86 IT 1976386**

(43) Date of publication of application:
**16.09.87 Bulletin 87/38**

(45) Publication of the grant of the patent:
**03.06.92 Bulletin 92/23**

(84) Designated Contracting States:
**AT BE CH DE ES FR GB LI NL SE**

(56) References cited:
**US-A- 3 135 788**
**US-A- 4 259 249**

(73) Proprietor: **ISTITUTO GUIDO DONEGANI S.p.A.**
**Via Caduti del Lavoro**
**I-28100 Novara(IT)**

(72) Inventor: **Francalanci, Franco**
**7, Via G. Ferraris**
**I-28100 Novara(IT)**
Inventor: **Ricci, Marco**
**5, Via Brescia**
**I-28100 Novara(IT)**
Inventor: **Cesti, Pietro**
**51, Via Torino**
**I-28069 Trecate Novara(IT)**
Inventor: **Venturello, Carlo**
**135, Via XXIII Marzo**
**I-28100 Novara(IT)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**W-8000 München 40(DE)**

**EP 0 237 080 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for the biotechnological preparation of L(-)-carnitine chloride having formula (I):

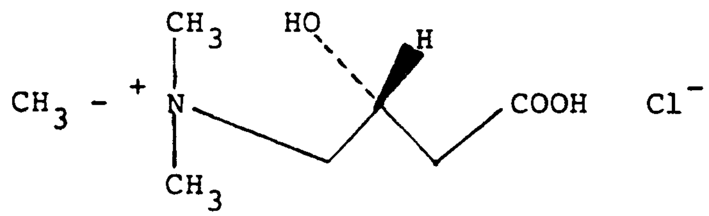

(I)

More particularly, the present invention relates to a process for the preparation of L(-)-carnitine chloride, starting from esters of R(+)-3,4-epoxybutyric acid, that are, in turn, obtained by asymmetrical enzymatic hydrolysis of particular esters of racemic 3,4-epoxybutyric acid.

The R(+) forms of said esters of 3,4-epoxybutyric acid which are used as intermeidates in the process of the present invention are novel compounds and as such form part of the invention.

As known, carnitine (also defined as β-hydroxy-trimethylaminobutyric acid) has a centre of asymmetry in the β-position and, therefore, two stereoisomers may exist, usually defined as D form and L form optical antipodes or optical enantiomers.

L(-)-carnitine obtained in the form of chloride according to the present invention, takes a prominent part in the field of the human metabolism and in the transfer of fatty acids. D(+)-carnitine, on the contrary, is an inhibiting agent competitive with respect to L(-)-carnitine-acyltransferase and may cause the lowering of the level of L(-)-carnitine present in the cardiac tissue, see Fritz, I.B., Schultz, S.K. J. Biol. Chem. (1965) 240 2188; Roe, C.R., Bohan T.P., (Lancet 1982) 1411.

The most ordinary therapeutic uses of L(-)-carnitine are, therefore, as eutrophic agent and as cardioprotecting agent for the treatment of myocardic ischemias, angina pectoris, and myocardium sclerosis.

Some processes are known for the carnitine synthesis, most of them, however, lead to carnitine in the racemic form; therefore, an operating stage is required, aiming at separating the racemic mixture into the two single optical antipodes. Therefore, aforesaid processes require expensive reactants, that are optically active, such as, for instance, dibenzoyltartaric acid, camphoric acid, mandelic acid etc. Moreover, they require a careful control of the reaction conditions and make several crystallization stages necessary; therefore they prove to be complicated and consequently burdensome from the economical and operating point of view, in particular from an industrial point of view (see European Patent Application EP 141.408; French Patent 1.466.696 and British Patent GB.- A - 2.131.049).

A process was also described for the L(-)-carnitine synthesis starting from an optically active compound such as D-mannitol (see European Patent Application EP 60.595). Such process, although it does not comprise any resolution stage, requires, however, a great deal of steps and the use of expensive and potentially dangerous reactants such as lead tetraacetate.

Some microbiologic processes are also known, leading to L(-)-carnitine starting from prochiralic substrates, such as alkyl chloroacetoacetates, crotonbetaines, or butyrobetaines (see Belgian Patent BE 898.396, European Patent Application EP 122.794; French Patent Application FR 2.485.564).

Such processes present the drawback to require bulky reaction volumes and are characterized by low yields and by difficulties in purifying the products.

Therefore the necessity was felt to have a process at disposal that could be carried out industrially, allowing the preparation of L(-)-carnitine in a simple, efficient and economic way.

Therefore an object of present invention is to make the preparation of L(-)-carnitine chloride possible according to a process simple from an operational point of view. and advantageous from an industrial point of view.

Another object is to provide a new class of intermediate compounds consisting of the enantiomeric R-(+) forms of particular esters of 3,4-epoxybutyric acid.

The Applicant has now found that these and still other objects may be reached by means of a biotechnological process based on the resolution of the optical R(+) isomers of particular racemic esters of 3,4-epoxybutyric acid, which resolution is carried out by enzymatic asymmetrical hydrolysis of said particular esters; from said R(+) forms or from their corresponding chlorohydrines, L(-)-carnitine chloride is then obtained by reaction with trimethylamine hydrochloride or with trimethylamine, and subsequent acid

hydrolysis (HCl).

In practice, in aforesaid enzymatic hydrolysis use is made of selected specific enzymes or of microrganisms producing said enzymes, which are capable of hydrolyzing, in a selective way (asymmetric hydrolysis), enantiomer S(-) of aforesaid recemic ester.

The corresponding enantiomeric R(+) forms of the particular racemic esters of 3,4-epoxybutyric acid obtained according to the process of the present invention are per se new intermediate compounds, to which the present invention is directed as well, according to a particular aspect of its.

Therefore an object of the present invention is to provide a process for the biotechnological preparation of L(-)-carnitine chloride, having formula (I), as hereinbefore defined, which process consists in:

a) reacting a racemic ester of (R,S)-3,4-epoxybutyric acid having formula:

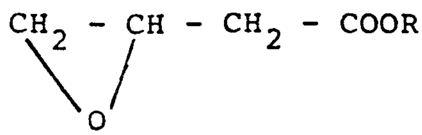 (II)

wherein R represents a $C_1$-$C_{10}$ alkyl group or a benzyl group, with an enzyme or with a microorganism producing said enzyme, capable of hydrolyzing asymmetrically and in a prevalent way enantiomer S(-), under pH conditions controlled by means of alkalis and in separating said enantiomer S(-) according to known techniques from non-reacted ester (II) which is substantially in the R(+) form;

b) reacting the R(+) form of ester (II), thus obtained, optionally converted, according to known techniques into its corresponding chlorohydrin with trimethylamine hydrochloride, or with trimethylamine itself, respectively, to obtain an ester having formula (IV)

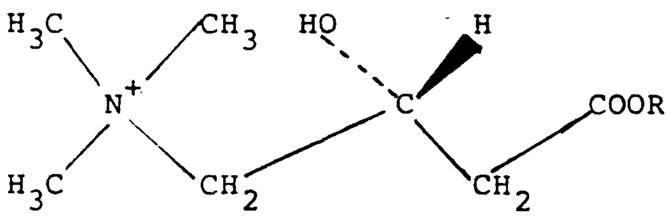 $Cl^-$ (IV)

wherein R has aforesaid meaning, and

c) hydrolyzing said ester having formula, (IV), in the presence of HCl to obtain L(-)-carnitine chloride having formula (I).

The intermediate compounds having formula (II), in their R(+) form, separated from the corresponding racemic compounds, according to the present invention, are not be described in literature and are, therefore, per se new compounds.

In fact, the resolution of such racemic esters having formula (II) into their enantiomers cannot be carried out easily by employing the usual methods, whereas, on the contrary, this is made possible according to the present invention.

The process may be represented schematically as follows:

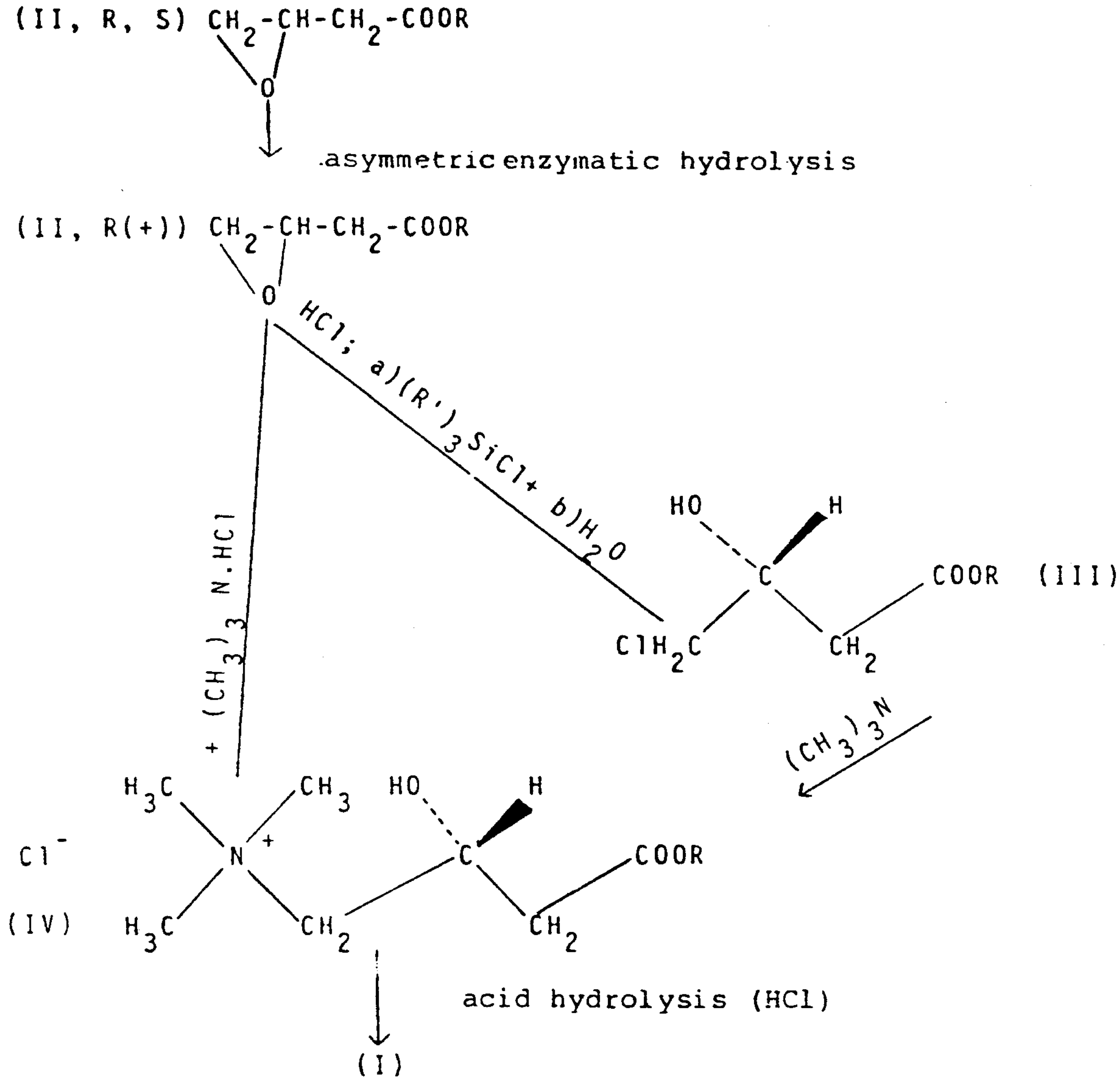

(II, R, S)
(II, R(+))
(III)
(IV)
(I)

wherein R has aforesaid meaning and R' represents a $C_1$-$C_5$ alkyl group.

The racemic esters having formula (II) are per se known compounds and may be prepared according to usual processes, such as, for instance:

1) 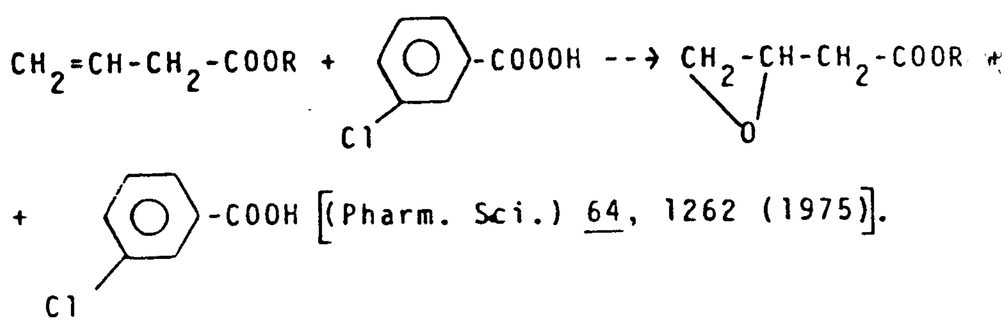
[(Pharm. Sci.) 64, 1262 (1975)].

2) 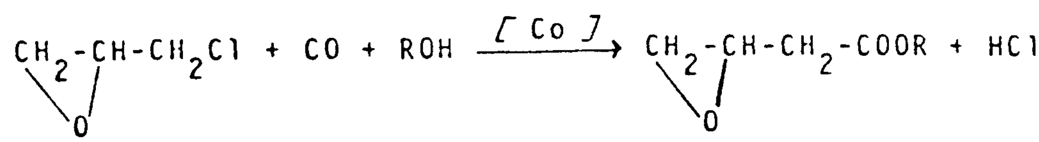


(J.Org.Chem.32,3888).

As above mentioned, the racemic esters having formula (II), are reacted with enzymes which may be produced by microorganisms or may be of animal origin, and which are capable of hydrolyzing selectively enantiomer S(-), letting the enantiomer in the R(+) form substantially unaltered (asymmetric hydrolysis).

To this purpose use may be made of hydrolytic enzymes of different origins which may be found on the market; among them, the following ones, as defined hereinafter, proved to be particularly active:

| ENZYME | ORIGIN | PRODUCER |
|---|---|---|
| Steapsin | swine pancreas | SIGMA Chem. Co. St. Louis - USA |
| Pancreatin | " " | UNIBIOS - Trecate (Italy) |
| Lipase from Candida cylindracea | Candida cylindracea | SIGMA Chem. Co. St. Louis - USA |
| Esterase from swine liver | swine liver | SIGMA Chem. Co.St. Louis - USA. |

As above mentioned, use may be made of microorganisms producing hydrolytic enzymes as well.

To this purpose use may be made of all the microornisms, provided they may produce enzymes which are capable of hydrolyzing asymmetrically the racemic esters having formula (II).

Among these microorganisms the following ones proved to be particularly efficient:

| | | |
|---|---|---|
| Pseudomonas Fragi | IFO | 3458 |
| Bacillus subtilis | ATCC | 6633 |
| Rodotorula minuta | IFO | 0879 |
| Candida cylindracea | ATCC | 14830 |
| Arthrobacter simplex | IFO | 3530. |

The first reaction of asymmetric hydrolysis of the racemic esters haying formula (II) is carried out by stirring strongly the mixture consisting of the racemic fester and of an aqueous solution of the raw or purified enzyme. Alternatively, the aqueous solution of the enzyme may replaced with the culture broth containing the microorganism, or with its filtrate or its concentrate or with suspensions of the microorganism cells as well.

Alternative, the enzyme may be used as immobilized on substrates of different nature, selected according to the prior art concerning this field. Use is made of enzyme amounts ranging between about 0.03% and 10% by weight based on the racemic ester (II).

The hydrolysis according to the invention may be carried out at a temperature ranging between about 5°C and 60°C, preferably between 10°C and 30°C, keeping the pH values between 5 and 9, preferably between 6 and 8, as the enzymes show their highest activity at these values.

The pH of the reaction medium is kept constant, by using a sodium and/or potassium buffer solution, by neutralization of the formed acidity by means of a mineral base such as, for instance, NaOH, KOH, LiOH, $CaCO_3$, etc.

The concentration of starting racemic ester (II) in the reaction mixture may range between 1% and 20% by weight. The reaction time of asymmetric hydrolysis may range between about 5 and 72 hours, according to the specific activity of the employed enzyme or to the desired conversion.

When the reaction of asymmetric hydrolysis is over, non-reacted ester (II), rich in isomer R(+), may be extracted from the reaction mixture by using a solvent immiscible with water such as, for instance, methylene chloride, toluene, ligroine, ethyl ether etc. The ester, thus obtained, may be purified according to usual techniques such as, for instance, distillation, column chromatography.

Epoxyester (II) recovered from the asymmetric hydrolysis, substantially in the R(+) form is reacted with trimethylamine hydrochloride to obtain an ester having formula (IV). According to a working practical way, the reaction is carried out by stirring a solution consisting of epoxyester (II), trimethylamine hydrochloride and a $C_1$-$C_4$ aliphatic (hydro)-alcoholic solvent. Said reaction may be carried out at a temperature ranging between about 10°C and 80°C, preferably between 20°C and 60°C and the reaction time may range between about 1 and 120 hours, according to the selected temperature. The concentration of epoxyester R-(+) (II) in the reaction mixture may range between about 10% and 60% and, preferably, between 20% and 50% by weight. The amount of used trimethylamine hydrochloride, may range between about 0.3 and 1 mole per mole of epoxyester R(+) (II) and the preferred value is 0.5 mole per mole. At the end of the reaction, the (hydro)-alcoholic solvent is distilled and the residue is treated with water and washed with a solvent immixible with water, such as, for instance, methylene chloride, ethyl ether etc. At the end, the water is removed by distillation, thereby obtaining the ester having formula (IV). Finally said ester is converted into L(-)-carnitine chloride by acid hydrolysis, carried out in aqueous hydrochloric acid, at a temperature ranging between about 15°C and 100°C, preferably between 80°C and 100°C, for reaction times ranging between about 1 and 20 hours, according to the selected temperature. The hydrochloric acid concentration may range between 5% and 37%, while its amount may range between about 1 and 10 moles per mole of ester having formula (IV).

When the hydrolysis reaction is over, the solution is evaporated at reduced pressure and the residue is crystalized, according to known techniques, thereby obtaining L(-)-carniti ne chloride having formula (I).

As above described, the ester having formula (IV) may be obtained according to the present invention as follows as well. Epoxyester (II), recovered from the reaction of enzymatic asymmetric hydrolysis, substantially in the R(+) form, is converted into the corresponding chlorohydrin having formula (III) by reaction with aqueous hydrochloric acid. The reaction is carried out suitably by dripping concentrated aqueous hydrochloric acid into a solution of epoxyester R(+) (II) in tetrahydrofuran (or in an ether solvent miscible with water), at a temperature ranging between about 0°C and 30°C and preferably between 0°C and 10°C. The reaction time may range between 1 and 4 hours according to the selected conditions. The amount of hydrochloric acid may range between about 1 and 2 moles per mole of epoxyester R(+) (II) and preferably between 1.0 and 1.1 moles per mole. At the end of the reaction, the mixture is neutralized by means of a base, such as, for instance, $Na_2CO_3$, then it is saturated with $Na_2SO_4$ or NaCl or with $Na_2CO_3$ itself and extracted by means of a solvent immiscible with water, such as, for instance, methylene chloride or ethyl ether. The solvent removal by distillation results in the raw chlorohydrin having formula (III). Then said chlorohydrin is converted into the ester having formula (IV) by reaction with trimethylamine in an alcoholic, aqueous-alcoholic medium or simply in water. The reaction is carried out suitably by adding a trimethylamine solution to chlorohydrin (III) and stirring the resulting mixture at a temperature ranging between about 20°C and 100°C, and preferably between 80°C and 90°C, for a period of time ranging between 1 and 20 hours, according to the selected temperature. Water, $C_1$-$C_4$ alcohols such as, for instance, methanol, ethanol or mixtures thereof are suitable as solvents for trimethylamine. The concentration of trimethylamine solution may range between about 5% and 33%, and preferably between about 25% and 33%, while the amine amount may range between about 1 and 10 moles per mole of chlorohydrin (III) and preferably between 2-3 moles per mole. At the end of the reaction trimethylamine in excess and solvent are removed by distillation; thus one obtains the raw ester having formula (IV), that is hydrolyzed, as described hereinbefore to obtain L(-)-carnitine.

The chlorohydrin having formula (III), in turn, may be obtained alternatively by reaction with a chlorosilane compound having formula $(R')_3$ SiCl wherein R' = a $C_1$-$C_5$ alkyl. In particular, the epoxyester recovered from the reaction of asymmetrical enzymatic hydrolysis, substantially in the R(+) form, is converted into the corresponding chlorohydrin having formula (III) by reaction with chlorotrimethylsilane or in general with a chloro-($C_1$-$C_5$)trialkylsilane and subsequent hydrolysis with water.

Ester R(+) (II) is dissolved in an excess of chlorosilanic compound substantially at room temperature and reacted about over a day.

At the end, after evaporation of silane in excess, the residue is treated with hydroalcoholic (ethanol) HCl and dried again, thereby obtaining the raw chlorohydrin having formula (III). Then it is treated as described hereinbefore.

From the obtained L(-)-carnitine chloride, by means of usual procedures, other derivatives of L(-)-carnitine, e.g. a salt of inorganic acids (HBr etc.) or organic acids(oxalic acid etc.) or its internal salt, may be

obtained.

A few examples will be given hereinafter by way of illustration but not of limitation of the invention.

The following abbreviations were used:

- $Eu(hfc)_3$ = europium tris [3-(heptafluoropropylhydroxymethylene)-d-camphorate].
- e. e. = enantiomeric excess.
- a. s. = as such.

The e. e. relating to R(+)-3,4-epoxybutyrates having formula (II) were determined by N.M.R. analysis at 300 MHz, in the presence of $Eu(hfc)_3$ (0.05 moles per mole of ester).

EXAMPLE 1

Stage a)

45 ml of a buffer solution consisting of Na and K phosphate at pH = 7 , 10 g of isobutyl (R,S)-3,4-epoxybutyrate (63.3 mmoles) and 640 mg of steapsin enzyme /lipase coming from swine pancreas, sold by the firm SIGMA Chem. Co., USA, having a protein content of 35% and an activity equal to 35-70 units per mg of protein) were added to 45 ml of a 0.1 M solution of KCl. The mixture was stirred strongly at 20°C and the pH was kept constant at value 7 by addition of an aqueous solution of 5N NaOH. After 22 hours (conversion of 3,4-epoxybutyrate equal to 65%), the residual ester was recovered from the reaction mixture by extraction with methylene chloride and afterwards purified by distillation. Thus one recovered 3.3 g of isobutyl R(+)-3,4-epoxybutyrate: $[\alpha]_D^{20}$ = + 9.900 (a.s.); $^1$H-NMR ($CDCl_3$) : 50.94 (d, 6H), 1.87-2.03 (m, 1H), 2.50-2.66 (m, 3H), 2.82-2.88 (m, 1H), 3.27-3.34 (m, 1H), 3.92 (d, 2H). NMR analysis at 300 MHz, in the presence of Eu $(hfc)_3$ (0.05 moles per mole of ester) showed an e. e. ≧ 90°C.

Stages b) + c)

Isobutyl R(+)-3,4-epoxybutyrate (3.3 g; 20.9 mmoles) was dissolved in 4 ml of methanol. 1 g of trimethylamine hydrochloride (10.46 mmoles) was added to the resulting solution and the whole was brought to 45°C for two hours under strong stirring. At the end methanol was removed by distillation and the residue was treated with water and ether. The phases were separated and the organic one was washed with water, which was then collected to the aqueous phase. The latter was washed with ether and concentrated, then 8 ml of concentrated aqueous hydrochloric acid were added to it. The solution was reflux heated over two hours and then evaporated at reduced pressure (about 20 mmHg). After removal of water traces by azeotropic distillation with ter-butyl alcohol, 1.19 g of L(-)-carnitine chloride were obtained, : $[\alpha]_D^{20}$ = -21.2°C (c = 1, $H_2O$); e. e. 90%.

EXAMPLE 2

Stage a)

One operated as in example 1, while replacing, however, isobutyl (R,S)-3,4-epoxybutyrate with 10 g of n-butyl (R,S)-3,4-epoxybutyrate (63.3 mmoles). After 26 hours (ester conversion equal to 60%), by extraction with methylene chloride and subsequent distillation, 3.8 g n-butyl R(+)-3,4-epoxybutyrate were recovered : $[\alpha]_D^{20}$ = 8.10° (a.s.); $^1$H-NMR ($CDCl_3$): :0.94 (t. 3H), 1.32-1.47 (m, 2H), 1.58-1.70 (m, 2H), 2.53--2.60 (m, 3H), 2.82-2.88 (m, 1H), 3.27-3.35 (m, 1H), 4.13 (t. 2H).

NMR analysis at 300 MHz, in the presence of $Eu(hfc)_3$, showed an e. e. ≧ 90%.

Stages b) + c)

One operated as in example 1, while replacing, however, isobutyl R(+)-3,4-epoxybutyrate with 3.8 g of n-butyl R(+)-3,4-epoxybutyrate (24.05 mmoles) and using 1.15 g of trymethylamine hydrochloride (12.0 mmoles). 1.75 g of L(-)-carnitine chloride were obtained; $[\alpha]_D^{20}$ = - 21.3° (c = 1, $H_2O$); e. e. 90%.

EXAMPLES 3-8

One operated as in example 1, while making, however, the changes indicated on Table 1. The obtained results are indicated on Table 1 as well.

EXAMPLE 9

Stage a)

The content of a slant of Arthrobacter simplex (IFO 3530) was inoculated in 50 ml of "Nutrient Broth" (nutrient broth manufactured by the firm Oxoid Ltd. U.K.) and kept under stirring at 200 rounds per minute, at 37°C, over 18 hours, in a 250 ml flask. Then four ml of this medium were inoculated in 100 ml of "Nutrient Broth" and kept under stirring at 200 rounds per minute, at 37°C, in a 500 ml flask.

After 12 hours, one added to the resulting mixture 50 ml of a,buffer solution consisting of Na and K phosphate at pH = 7 and 5 g of isobutyl (R,S)-3,4-epoxybutyrate and the whole was kept under stirring at 20°C over 46 hours. At the end the mixture was extracted with methylene chloride, the solvent was evaporated and the residual isobutyl R(+)-3,4-epoxybutyrate was purified by chromatography on silica column. Thus one obtained 2.25 g of isobutyl R(+)-3,4-epoxybutyrate having an e. e. = 75%.

Stages b) and c)

One operated as in example 1, thereby obtaining 0.84 g of L(-)-carnitine chloride having an e. e. = 75%.

EXAMPLE 10

One operated as in example 9, with the difference that the used microorganism was Pseudomonas fragi (IFO 3458), thereby obtaining 2.35 g of isobutyl R(+)-3,4-epoxybutyrate having an e.e. = 56%. Then from this ester one obtained 0.82 g of L(-)-carnitine chloride having an e.e. = 55%.

EXAMPLE 11

One operated as in example 9, with the difference that the used microorganism was Bacillus subtilis - (ATCC 6633), thereby obtaining 2.85 g of isobutyl R(+)-3,4-epoxybutyrate having an e.e. = 52%. Then from this ester one obtained 1.0 g of L(-)-carnitine chloride having an e.e. = 52%.

EXAMPLE 12

Stage a)

The content of a slant of Rodotorula minuta (IFO 0879) was inoculated in 50 ml of a culture medium having the following composition: 0.3% of yeast extract by the firm Oxoid Ltd. U. K., 1% of peptone, Oxoid, 2% of glucose.

The medium was put into a 250 ml flask and kept under stirring at 160 rounds per minute, at 28°C, over 18 hours. Then four ml of this medium were drawn and inoculated in 100 ml of a culture medium having the same composition as the one described hereinbefore; then 0.5 g of calcium carbonate were added and the whole was kept under stirring at 16 rounds per minute, at 28°C. After 24 hours, 5 g of isobutyl (R,S)-3,4-epoxybutyrate were added to the resulting mixture and the whole was kept under stirring over 72 hours at 20°C. At the end, the mixture was extracted by means of methylene chloride, the solvent was evaporated and the residual isobutyl R(+)-3,4-epoxybutyrate was purified by chromatography on silica column. Thus one obtained 3.1 g of isobutyl R(+)-3,4-epoxybutyrate having an e.e. = 27%.

Stages b) + c)

One operated as in example 1, thereby obtaining 1.16 g of L(-)-carnitine chloride having an e.e. = 27%.

EXAMPLE 13

One operated as in example 12, with the difference that the used microorganism was Candida cylindracea (ATCC 14830), thereby obtaining 2.75 g isobutyl R(+)-3,4-epoxybutyrate having an e.e. = 47%. Then from this ester one obtained 0.93 g of L(-)-carnitine chloride having an e.e. = 45%.

EXAMPLE 14

Stage a)

One operated as in example 1, thereby obtaining 3.2 g of isobutyl R(+)-3,4-epoxybutyrate having an e.e. = 90%.

Stages b) + c)

Said isobutyl R(+)-3,4-epoxybutyrate was dissolved in 40 ml of tetrahydrofuran. 1.66 ml of concentrated aqueous hydrochloric acid were dripped over one hour, into the resulting solution, cooled down to a temperature ranging between 0° and 5°C, by means of an ice bath and kept under strong stirring. At the end of the addition the temperature was let rise up to 20°C and the stirring was kept on for one further hour. Then the solution was saturated with $Na_2CO_3$, concentrated and extracted by means of ether. Then the ethereal extract was dried on $Na_2SO_4$, filtered and evaporated under reduced pressure, thereby obtaining 3.26 g of an oil that, according to the analysis, proved to be isobutyl R(+)-4-chloro-3-hydroxybutyrate (III) with a gaschromatographic titre of 94%. Then said oil was dissolved in 8 ml of ethanol and in 8 ml of an aqueous solution of 5.19 M trimethylamine and the resulting solution was reflux heated, under strong stirring, over two hours. At the end the solution was evaporated, under reduced pressure and the residue was treated with 30 ml of aqueous concentrated hydrochloric acid and reflux heated over two hour under stirring. At the end the solution was evaporated under reduced pressure again and, after removal of water traces by azeotropic distillation with ter-butyl alcohol, 1.13 g of L(-)-carnitine chloride were obtained, $[\alpha]_D^{20}$ = -15.4°; e.e. = 65%.

EXAMPLE 15

Stage a)

One operated as in example 1 thereby obtaining 3.2 g of isobutyl R(+)-3,4-epoxybutyrate having an e.e. = 90%.

Stage b) + c)

Said isobutyl R(+)-3,4-epoxybutyrate was dissolved in 12 ml of chlorotrimethylsilane and the resulting solution was stirred at room temperature over 20 hours. Then chloromethylsilane in excess was removed by distillation and the residue was treated with 30 ml of methanol and 1 ml of aqueous hydrochloric acid at 10% and kept under stirring, at room temperature, for 10 minutes. Then the solution was evaporated at reduced pressure and the residue was treated with ether and washed with water. The ethereal phase was dried on $Na_2SO_4$,filtered and evaporated; thus one obtained 3.84 g of isobutyl R(+)-4-chloro-3-hydroxybutyrate (III) with a gaschromatographic titre of 90%. Then one operated as in example 14, thereby obtaining 1.20 g of L(-)-carnitine chloride, $[\alpha]_D^{20}$ = -14.3°; e.e. = 60%.

| Example No | R | ENZYME (amount) | TIME (hours) | ESTER CONV. % | CARACTERISTICS R(+)-3,4-epoxybutyrate NMR, δ | $[\alpha]^{20}_{D}$ | e.e. | g. of CARNITINE CHLORIDE | e.e. CARNITINE CHLORIDE |
|---|---|---|---|---|---|---|---|---|---|
| | | STAGE a) | | | | | | | |
| 3 | iso-butyl | Lipase from Candida Cylindracea (400 mg) | 39 | 57% | 0,94 d (6H); 1,87-2,03 m (1H) 2,50-2,66 m (3H); 2,82-2,88 m (1H); 3,27-3,34 m (1H); 3,92 d (2H) | +8.14 | 74% | 1.39 | 74% |
| 4 | iso-butyl | Esterase from swine liver (3.5 mg) | 58 | 52% | " | +6.82 | 62% | 1.60 | 61% |
| 5 | iso-butyl | Pancreatin (240 mg) | 21 | 60% | " | +8.25 | 75% | 1.30 | 74% |
| 6 | n-octyl | Steapsin (500 mg) | 24 | 60% | 0,88 t (3H); 1,16-1,42 m (10H); 1,58-1,71 m (2H); 2,53-2,61 m (3H); 2,82-2,88 m (1H); 3,24-3,33 m (1H); 4,12 t (2H) | +4,73 | ≥ 95% | 1.60 | 95% |
| 7 | methyl | Lipase from Candida Cylindracea (400 mg) | 48 | 60% | 2,54-2,60 m (3H); 2,83-2,88 m (1H); 3,26-3,34 m (1H) 3,74 s (3H) | n.d. | 50% | 1.41 | 50% |
| 8 | benzyl | Steapsin (500 mg) | 6.5 | 62% | 2,55-2,57 m (1H); 2,61-2,63 m (2H); 2,82-2,85 m (1H); 3,27-3,35 m (1H); 5,17 s (2H); 7,25-7,37 m (5H) | n.d. | 58% | 1.67 | 60% |

## Claims

**1.** A process for the preparation of L(-)-carnitine chloride having formula:

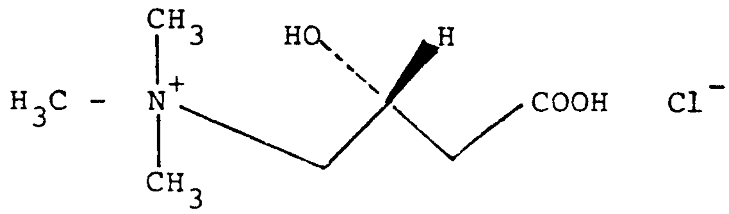 (I)

which comprises:

a) reacting a racemic ester of (R,S)-3,4-epoxybutyric acid having formula (II):

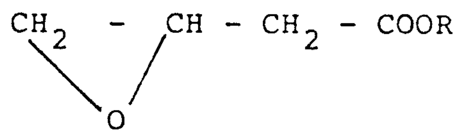 (II)

wherein R represents a $C_1$-$C_{10}$ alkyl group or a benzyl group, with an enzyme or a microorganism producing said enzyme, capable of hydrolyzing asymmetrically and in a prevalent way enantiomer S(-), under pH conditions controlled by means of alkalis and separating said enantiomer S(-) according to usual techniques from non-reacted ester (II) which is substantially present in the R(+) form;

b) reacting the R(+) form of ester (II) thus obtained, with trimethylamine hydrochloride or, after having converted ester (II) according to known techniques into its corresponding chlorohydrin, with trimethylamine to obtain an ester having formula

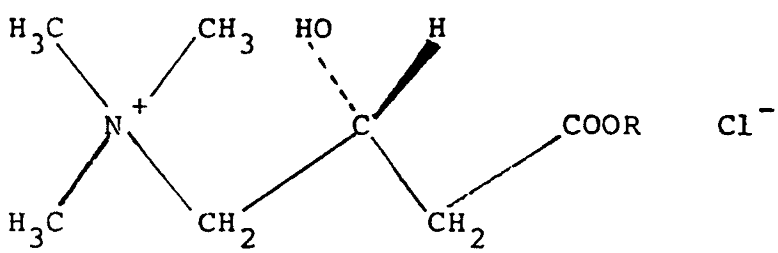 (IV)

wherein R has the aforesaid meaning and

c) hydrolyzing said ester having formula (IV) in the presecne of HCl to obtain L(-)-carnitine chloride having formula (I).

**2.** A process according to claim 1, wherein the asymmetrical hydrolysis of the racemic ester having formula (II) is carried out using an enzyme, preferably selected from steapsin, pancreatin, lipase from Candida Cylindracea and esterase from swine liver.

**3.** A process according to claim 1, wherein the asymmetrical hydrolysis of the racemic ester having formula (II) is carried out using an enzyme produced by microorganismus, preferably selected from Pseudomonas Fragi (IFO 3458), Bacillus subtilis (ATCC 6633), Rodotorula minuta (IFO 0879), Candida Cylindracea (ATCC 14830) and Arthrobacter simplex (IFO 3530).

**4.** A process according to claim 3, wherein the microorganism is used in the form of a culture broth, a filtrate, a concentrate or a suspension of its cells.

**5.** A process according to claim 2, wherein the enzyme is immobilized on a substrate.

**6.** A process according to any one of claims 1 to 5, wherein the asymmetrical hydrolysis of the racemic ester (II) is carried out by using the enzyme in an amount of from 0.03 to 10% by weight, based on the racemic ester (II).

**7.** A process according to any one of claims 1 to 6, wherein the asymmetrical hydrolysis of the racemic ester (II) is carried out at a temperature of from 10 to 30 ° C.

**8.** A process according to any one of claims 1 to 7, wherein the asymmetrical hydrolysis of the racemic ester (II) is carried out at a pH value of from 5 to 9, using a basic buffer solution or a mineral base.

**9.** A process according to any one of claims 1 to 8, wherein the concentration of the racemic ester (II) in the mixture for the asymmetrical hydrolysis ranges from 1 to 20% by weight.

**10.** A process according to any one of claims 1 to 9, wherein the R(+) ester having formula (II) is converted into the ester having formula (IV) by reaction with trimethylamine hydrochloride in an aliphatic $C_1$-$C_4$ (hydro)-alcoholicsolvent at a temperature of from 10 to 80 ° C with a molar ratio of $(CH_3)_3$ N.HCl:R(+) ester (II) of from 0.3:1 to 1:1.

**11.** A process according to any one of claims 1 to 10, wherein the R(+) ester having formula (II) is converted into the ester having formula (IV) by reaction with an at least equimolar amount of aqueous HCl in the presence of an organic ether solvent miscible with $H_2O$, preferably tetrahydrofuran, at a temperature of from 0 ° C to 30 ° C and subsequent treatment of the chlorohydrin of the R(+) ester (II) thus obtained with an at least equimolar amount of trimethylamine in a ($C_1$-$C_4$) (hydro)-alcoholic solvent or an aqueous medium at a temperature of from 20 to 100 ° C.

**12.** A process according to claim 11, wherein the R(+) ester having formula (II) is converted into its chlorohydrin derivative by reaction with a chloro-alkylsilane compound having formula $(R')_3$ SiCl, wherein R' represents a $C_1$-$C_5$ alkyl group.

**13.** A process according to any one of claims 1 to 12, wherein the acid hydrolysis of the ester having formula (IV) is carried out with an at least equimolar amount of aqueous HCl at a temperature of from 15 to 100 ° C.

**14.** The R(+) enantiomers of the esters of 3,4-epoxybutyric acid having formula:

R(+) -

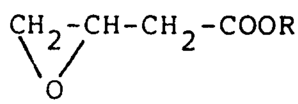

wherein R represents an alkyl group containing 1 to 10 carbon atoms or a benzyl group.

**15.** A process for the preparation of R(+) enantiomers of the esters of 3,4-epoxybutyric acid having formula (II) as defined in claim 1, which comprises reacting a racemic ester of formula (II) with an enzyme or a microorganism producing said enzyme, capable of hydrolyzing asymmetrically and in a prevalent way enantiomer S(-), under pH conditions controlled by means of alkalis and separating said enantiomer S(-) according to usual techniques from non-reacted ester (II) which is substantially present in the R(+) form.

**Revendications**

**1.** Un procédé de préparation de chlorure de L(-)-carnitine présentant la formule:

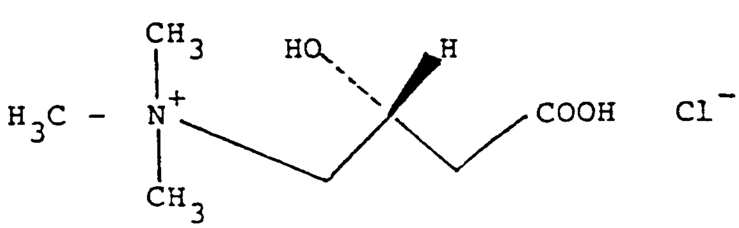

(I)

comprenant les étapes suivantes:

a) réaction d'un ester racémique de l'acide (R,S)-3,4-époxybutyrique répondant à la formule:

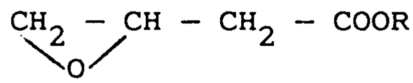

(II)

dans laquelle R représente un groupe alkyle en $C_1$ à $C_{10}$ ou un groupe benzyle, avec un enzyme ou avec un microorganisme secrétant cet enzyme capable d'hydrolyser asymétriquement et de façon prédominante les énantiomères S(-) dans des conditions de pH contrôlées par des bases et la séparation de cet énantiomère S(-) par des techniques connues en elles-mêmes de l'ester (II) n'ayant pas réagi qui se trouve sensiblement sous forme R(+);

b) réaction de la forme R(+) de l'ester (II) ainsi obtenu avec du chlorhydrate de triméthylamine ou, après avoir converti l'ester (II) selon des techniques connues en elles-mêmes, en sa forme chlorohydrine correspondante, avec de la triméthylamine, pour obtenir un ester répondant à la formule (IV):

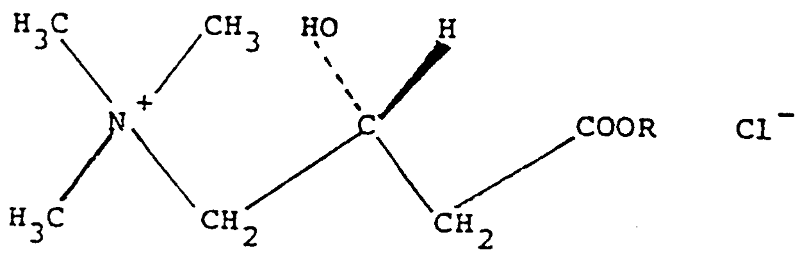

(IV)

dans laquelle R possède la signification ci-dessus, et

c) hydrolyse de cet ester répondant à la formule (IV) en présence de HCl pour obtenir le chlorure de L(-)-carnitine répondant à la formule (I).

**2.** Un procédé selon la revendication 1, caractérisé en ce que l'hydrolyse asymétrique de l'ester racémique de formule (II) est mise en oeuvre un utilisant un enzyme, choisi de préférence parmi: stéapsine, pancréatine, lipase de Candida cylindracea et estérase de foie de porc.

**3.** Un procédé selon la revendication 1, caractérisé en ce que l'hydrolyse asymétrique de l'ester racémique de formule (II) est mise en oeuvre un utilisant un enzyme secrété par un micro-organisme, choisi de préférence parmi: Pseudomonas Fragi (IFO 3458), Bacillus subtilis (ATCC 6633), Rodotorula minuta (IFO 0879), Candida cylindracea (ATCC 14830) et Arthrobacter simplex (IFO 3530).

**4.** Un procédé selon la revendication 3, caractérisé en ce que le micro-organisme est utilisé sous forme d'un milieu de culture, un filtrat, un concentré ou une suspension de ses cellules.

**5.** Un procédé selon la revendication 2, caractérisé en ce que l'enzyme est immobilisé sur un substrat.

**6.** Un procédé selon l'une quelconque des revendications 1 à 5, caractérisé en ce que l'hydrolyse asymétrique de l'ester racémique (II) est mise en oeuvre en utilisant l'enzyme en quantité comprise entre 0,3 et 10% en poids par rapport à l'ester racémique (II).

**7.** Un procédé selon l'une quelconque des revendications 1 à 6, caractérisé en ce que l'hydrolyse asymétrique de l'ester racémique (II) est mise en oeuvre à une température de 10 à 30 ° C.

**8.** Un procédé selon l'une quelconque des revendications 1 à 7, caractérisé en ce que l'hydrolyse asymétrique de l'ester (II) est mise en oeuvre à une valeur de pH de 5 à 9, en utilisant une solution tampon basique ou une base minérale.

**9.** Un procédé selon l'une quelconque des revendications 1 à 8, caractérisé en ce que la concentration de

l'ester racémique (II) dans le mélange soumis à l'hydrolyse asymétrique est comprise entre 1 et 20% en poids.

**10.** Un procédé selon l'une quelconque des revendications 1 à 9, caractérisé en ce que l'ester R(+) de formule (II) est converti en ester de formule (IV) par réaction avec le chlorhydrate de triméthylamine dans un solvant (hydro)-alcoolique aliphatique en $C_1$ à $C_4$ à une température de 10 à 80°C, le rapport molaire de $(CH_3)_3$ N.HCl:R(+) ester (II) étant compris entre 0,3/1 et 1/1.

**11.** Un procédé selon l'une quelconque des revendications 1 à 10, caractérisé en ce que l'ester R(+) de formule (II) est converti en ester de formule (IV) par réaction avec une quantité au moins équimolaire de HCl aqueux en présence d'un solvant à base d'éther organique miscible à l'eau, de préférence le tétrahydrofurane, à une température de 0 à 30°C et ensuite traitement de la forme chlorhydrine de l'ester R(+) (II) ainsi obtenue par une quantité au moins équimoléculaire de triméthylamine dans un solvant (hydro)-alcoolique (en $C_1$ à $C_4$) ou un milieu aqueux à une température de 20 à 100°C.

**12.** Un procédé selon la revendication 11, caractérisé en ce que l'ester R(+) de formule (II) est converti en sa chlorhydrine par réaction avec un dérivé de chloroalkylsilane répondant à la formule $(R')_3$ SiCl, dans laquelle R' représente un groupe alkyle en $C_1$ à $C_5$.

**13.** Un procédé selon l'une quelconque des revendications 1 à 12, caractérisé en ce que l'hydrolyse acide de l'ester de formule (IV) est mise en oeuvre en présence d'au moins une quantité équimolaire d'HCl aqueux à une température de 15 à 100°C.

**14.** Les énantiomères R(+) de esters d'acide 3,4-époxybutyrique répondant à la formule:

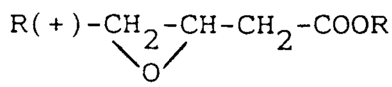

dans laquelle R représente un groupe alkyle contenant de 1 à 10 atomes de carbone ou un groupe benzyle.

**15.** Un procédé pour la préparation d'énantiomères R(+) des esters de l'acide 3,4-époxybutyrique présentant la formule (II) telle que définie dans la revendication 1, qui consiste à faire réagir un ester racémique de formule (II) avec un enzyme ou un micro-organisme secrétant cet enzyme, capable d'hydrolyser asymétriquement et de façon prédominante les énantiomères S(-) dans des conditions de pH contrôlées par des bases et la séparation de cet énantiomère S(-) par des techniques connues en elles-mêmes de l'ester (II) n'ayant pas réagi que se trouve sensiblement sous forme R(+).

## Patentansprüche

**1.** Verfahren zur Herstellung von L(-)-Carnitinchlorid der Formel:

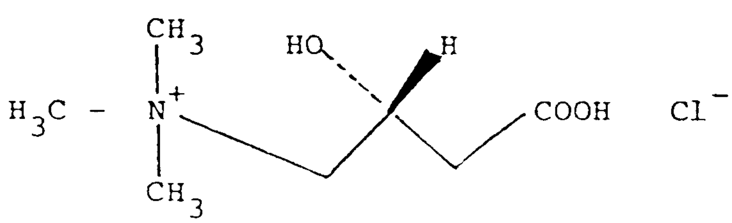

(I)

welches umfaßt:

a) das Umsetzen eines racemischen Esters von (R,S)-3,4-Epoxybuttersäure der Formel (II):

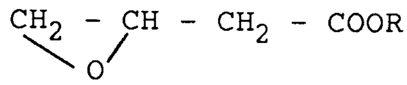 (II)

worin R eine $C_1$-$C_{10}$ Alkylgruppe oder eine Benzylgruppe darstellt, mit einem Enzym oder mit einem Mikroorganismus, der das Enzym erzeugt, das imstande ist, asymmetrisch und in bevorzugter Weise das S(-)-Enantiomer zu hydrolysieren, unter pH-Bedingungen, die mit Alkali kontrolliert werden, und die Abtrennung des S(-)-Enantiomers mit üblichen Methoden von nicht umgesetztem Ester (II), der im wesentlichen in der R(+)-Form vorliegt;

b) das Umsetzen der so erhaltenen R(+)-Form des Esters (II) mit Trimethylamin-hydrochlorid oder, nachdem der Ester (II) nach bekannten Methoden in das entsprechende Chlorhydrin überführt wurde, mit Trimethylamin, um einen Ester der Formel

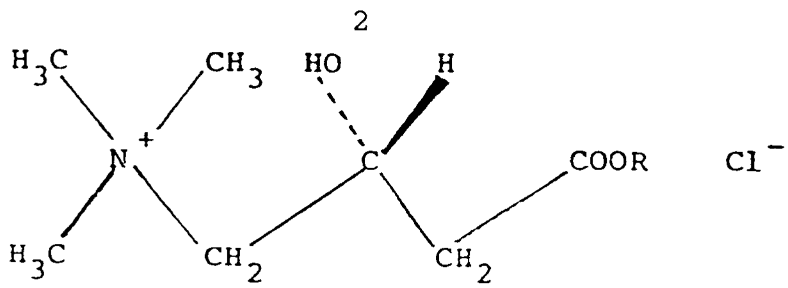 (IV)

zu erhalten, worin R die obige Bedeutung hat, und

c) die Hydrolyse des Esters der Formel (IV) in Gegenwart von HCl, um L(-)-Carnitinchlorid der Formel (I) zu erhalten.

**2.** Verfahren nach Anspruch 1, worin die asymmetrische Hydrolyse des racemischen Esters der Formel (II) unter Verwendung eines Enzyms ausgeführt wird, das vorzugsweise aus Steapsin, Pankreatin, Lipase von Candida Cylindracea und Esterase aus Schweineleber ausgewählt ist.

**3.** Verfahren nach Anspruch 1, worin die asymmetrische Hydrolyse des racemischen Esters der Formel (II) unter Verwendung eines Enzyms ausgeführt wird, das von einem Mikroorganismus erzeugt wird, der vorzugsweise aus Pseudomonas Fragi (IFO 3458), Bacillus subtilis (ATCC 6633), Rodotorula minuta - (IFO 0879), Candida Cylindracea (ATCC 14830) und Arthrobacter simplex (IFO 3530) ausgewählt ist.

**4.** Verfahren nach Anspruch 3, worin der Mikroorganismus in Form einer Kulturflüssigkeit, eines Filtrats, eines Konzentrats oder einer Suspension seiner Zellen verwendet wird.

**5.** Verfahren nach Anspruch 2, worin das Enzym auf einem Träger immobilisiert ist.

**6.** Verfahren nach irgendeinem der Ansprüche 1 bis 5, worin die asymmetrische Hydrolyse des racemischen Esters (II) unter Verwendung des Enzyms in einer Menge von 0,03 bis 10 Gew.-%, bezogen auf den racemischen Ester (II), durchgeführt wird.

**7.** Verfahren nach irgendeinem der Ansprüche 1 bis 6, worin die asymmetrische Hydrolyse des racemischen Esters (II) bei einer Temperatur von 10 bis 30°C durchgeführt wird.

**8.** Verfahren nach irgendeinem der Ansprüche 1 bis 7, worin die asymmetrische Hydrolyse des racemischen Esters (II) bei einem pH-Wert von 5 bis 9 unter Verwendung einer basischen Pufferlösung oder einer Mineralbase durchgeführt wird.

**9.** Verfahren nach irgendeinem der Ansprüche 1 bis 8, worin die Konzentration des racemischen Esters (II) in der Mischung für die asymmetrische Hydrolyse im Bereich von 1 bis 20 Gew.-% liegt.

**10.** Verfahren nach irgendeinem der Ansprüche 1 bis 9, worin der R(+)-Ester der Formel (II) in den Ester

der Formel (IV) überführt wird durch die Umsetzung mit Trimethylamin-hydrochlorid in einem aliphatischen $C_1$-$C_4$ (hydro)-alkoholischen Lösungsmittel bei einer Temperatur von 10 bis 80 °C in einem molaren Verhältnis von $(CH_3)_3N \cdot HCl$ : R(+)-Ester (II) von 0,3:1 bis 1:1.

**11.** Verfahren nach irgendeinem der Ansprüche 1 bis 10, worin der R(+)-Ester der Formel (II) in den Ester der Formel (IV) überführt wird durch die Umsetzung mit einer mindestens äquimolaren Menge wäßriger HCl in Gegenwart eines mit $H_2O$ mischbaren, organischen Ether-Lösungsmittels, vorzugsweise Tetrahydrofuran, bei einer Temperatur von 0 bis 30 °C und nachfolgender Behandlung des so erhaltenen Chlorhydrins des R(+)-Esters (II) mit einer mindestens äquimolaren Menge von Trimethylamin in einem ($C_1$-$C_4$) (hydro)-alkoholischen Lösungsmittel oder einem wäßrigen Medium bei einer Temperatur von 20 bis 100 °C.

**12.** Verfahren nach Anspruch 11, worin der R(+)-Ester der Formel (II) in sein Chlorhydrin-Derivat überführt wird durch Umsetzung mit einer Chloralkylsilan-verbindung der Formel $(R')_3SiCl$, worin R' eine $C_1$-$C_5$ Alkylgruppe bedeutet.

**13.** Verfahren nach irgendeinem der Ansprüche 1 bis 12, worin die Säurehydrolyse des Esters der Formel (IV) mit einer mindestens äquimolaren Menge wäßriger HCl bei einer Temperatur von 15 bis 100 °C durchgeführt wird.

**14.** R(+)-Enantiomere der Ester von 3,4-Epoxybuttersäure der Formel:

R(+) –

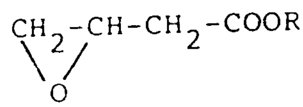

worin R eine Alkylgruppe mit 1 bis 10 Kohlenstoffatomen oder eine Benzylgruppe darstellt.

**15.** Verfahren zur Herstellung von R(+)-Enantiomeren der Ester von 3,4-Epoxybuttersäure der Formel (II) nach Anspruch 1, welches umfaßt die Umsetzung eines racemischen Esters der Formel (II) mit einem Enzym oder einem Mikroorganismus, der das Enzym erzeugt, das imstande ist, asymmetrisch und in bevorzugter Weise das S(-)-Enantiomer zu hydrolysieren, unter pH-Bedingungen, die mit Alkali kontrolliert werden, und die Abtrennung des S(-)-Enantiomers mit üblichen Methoden von nicht umgesetztem Ester (II), der im wesentlichen in der R(+)-Form vorliegt.